# EUROPEAN PATENT SPECIFICATION

(11) **EP 1 810 622 B1**
(45) Date of publication and mention of the grant of the patent: **05.12.2012**
(21) Application number: 07001214.1
(22) Date of filing: 19.01.2007
(51) Int. Cl.: A61B 17/122, A61B 17/12

(54) **Surgical hemostatic clip**
Hämostatische chirurgische Klammer
Pince chirurgicale hémostatique

(30) Priority: 23.01.2006 US 338911
(43) Date of publication of application: 25.07.2007
(73) Proprietor: Tyco Healthcare Group LP, Mansfield, MA 02048 (US)
(72) Inventor: Sorrentino, Greg, Wallingford, CT 06492 (US); Whitfield, Kenneth H., New Haven, CT 06511 (US)
(74) Representative: HOFFMANN EITLE

(56) References cited:
- EP-A- 1 602 335
- EP-A1- 0 287 275
- WO-A-02/11622
- WO-A-94/01044
- US-A- 4 188 953
- US-A- 4 702 247
- US-B1- 6 610 073

## Description

### BACKGROUND

### 1. Technical Field

This invention relates to surgical clips, and more particularly, to hemostatic surgical clips for application to blood vessels or body tissue.

### 2. Background

Ligation or occlusion of veins, arteries or blood vessels is a critical part of some surgical procedures. A severed vessel requires closure on both sides of a severance site before actual cutting takes place using another cutting instrument or knife.

Surgeons have used thread or suture material to tie a vessel prior to severing the vessel. This procedure was often time-consuming and required great surgeon dexterity. In many instances, the assistance of a nurse or attending surgeon was necessary to perform this procedure to perfect grasping and tying the vessel, then repeatedly testing the vessel to ensure closure. If complete closure of the vessel was not achieved using the suture material, then the sequence was repeated.

Surgical clips and hemostatic surgical clip appliers greatly enhances the art of vessel occlusion. Surgical clips are now commonly used for vessel ligation and occlusion. Examples of surgical hemostatic clips are described in U.S. Pat. Nos. 6,610,073, 5,501,693 , 5,171,253; 5,171,252; 5,100,420; 5,084,057 ; 4,971,198; 4,844,066 ; 4,799,481 ; 4,702,247; 4,414,721; 4,188,953; 4,146,130 ; 3,867,944 ; and 3,363,628, EP 0 287 275, WO 02/11622, EP 1 602 335 and WO 94/01044.

Certain factors are important to the performance of a surgical hemostatic clip and to achieve proper tissue exudation and occlusion. The clip should not slip or become dislodged from a vessel after it has been applied. If the clip is not securely positioned, blood or other bodily fluid may begin flowing into the surgical site through the unclamped vessel. A surgeon must locate and reclamp the vessel. Depending upon the type and location of the surgery, reclamping the vessel may be difficult, and reduce an overall productivity of the procedure. A clip should fully and completely close about a vein, artery, vessel or other conduit and completely stop the flow of blood or fluid therethrough. A clip that does not completely occlude the blood or fluid flow may have to be removed thus requiring application of a second clip.

Some surgical hemostatic clips are U-shaped or V-shaped. These clips have a pair of legs joined at one end by an apex or crown and spaced apart at the opposed ends to define a gap between the legs. The desired vessel is introduced in the gap and the legs are compressed. The clip thus occludes the vessel using the legs.

The legs have surfaces that contact tissue. These "tissue gripping surfaces" of the hemostatic clip can be made in a manner to improve the occluding functions of the hemostatic clip. The surfaces can also restrict dislocation of the hemostatic clip after it has been applied to the target blood vessel. However, often legs have a relatively small tissue gripping surface. Care must be taken when designing such tissue gripping surfaces to ensure that the most productive use of the relatively small tissue gripping surface is made to accomplish the occlusion. A significant aspect of the tissue gripping surfaces is this retention of the hemostatic clip on the tissue. Accordingly, there is a need in the art for an improved surgical hemostatic clip to provide an optimum vessel occlusion and optimal clip retention on tissue during a surgical procedure.

### SUMMARY

The present invention provides a surgical clip as defined in claim 1. According to an embodiment, there is provided a surgical clip for a clip applier. The surgical clip has a first leg connected to a second leg at a first location. The first leg is separated from the second leg by a predetermined distance at a second location spaced from the first location. The first leg has a first distal end and the second leg has a second distal end. The first leg has a tissue gripping region and the second leg has a second tissue gripping region. The first tissue griping region has a first recess on the first leg, and the second tissue gripping region has a second recess on the second leg. When the clip is compressed the first recess is compressed toward the second recess. The first recess and the second recess overlap to form a two dimensional polygonal pattern with one another. The first recess is compressed toward the second recess and the first and the second recess form a two dimensional diamond shaped pattern. The first recess consists of an apex portion and a first and a second distal leg portion extending from the apex portion. The surgical clip has the second recess with an apex portion and a first and a second distal leg extending from the apex portion. The first clip leg is compressed to the second clip leg and the apex portion of the first clip leg and the apex portion of the second leg point toward opposite directions.

According to another embodiment, the first leg has a plurality of first recesses and the second leg has a plurality of second recesses with substantially the same orientation. Each first recess registers with another of the second recesses when the first clip leg is compressed toward the second clip leg.

According to another embodiment, the clip has a grip feature on an outer clip leg surface.

According to another aspect of the present invention, the surgical clip is a hemostatic clip and is made from a material selected from the group consisting of stainless steel, a polymer, titanium, a biocompatible material, and any combinations thereof.

According to another aspect of the present invention, the first recess and the second recess or contained laterally within the respective first and second tissue gripping surfaces.

According to yet a further aspect of the present disclosure there is provided, but not claimed, a method of treating a hemostatic clip. The method has the steps of heating the clip in a vacuum to a desired temperature. The temperature is in a range of 1,275 degrees Fahrenheit. The method has the steps of holding the clip at the desired temperature for a desired period of time and soaking the clip in an inert gas for a desired period of time. The method has the steps of exposing the clip in a gas and cooling the clip to room temperature at a slow cooling rate.

According to yet a further aspect of the present disclosure there is provided, but not claimed, a method of treating a hemostatic clip where the method has the step of soaking the clip in an argon gas.

According to yet a further aspect of the present disclosure, the method has the step of the clipbeing exposed to argon gas.

According to yet a further aspect of the present disclosure, the clip is a biocompatible titanium and the clip is soaked for about one hour.

According to yet a further aspect of the present disclosure there is provided, but not claimed, a method of annealing a hemostatic clip comprising heating the clip in a vacuum to a desired temperature, wherein the temperature is in a range that includes 1,250 degrees Fahrenheit (677°C) to 1,275 degrees Fahrenheit (690.6°C) and holding the clip in the vacuum at the desired temperature for a desired period of time. The method also has the steps of soaking the clip for a desired period of time in the inert gas with the period of time being about one hour and exposing the clip in an argon gas to control a microstructure of the clip and slow cooling the clip to ambient temperature. The clip is biocompatible titanium.

According to yet a further aspect of the present disclosure the method has the step of machining a first plurality of recesses having a first apex in a first leg of the clip, and machining a second plurality of recesses having a second apex in a second leg of the clip. When the clip is compressed the second plurality of recesses are in registration with the first plurality of recesses. The first apex is disposed one hundred and eighty degrees from the second apex to form a compressed diamond shaped pattern.

### BRIEF DESCRIPTION OF THE DRAWINGS

FIG. 1 is a front view of a surgical clip according to the present disclosure;

FIG. 2 is a perspective view of the clip of Fig. 1 having a generally non-limiting "V" shape;

FIG. 3 is a top elevation view of the clip of FIG. 2 which is not an embodiment of the present invention, showing a number of recesses in a first predetermined gripping pattern on the first leg and a number of recesses in a second predetermined gripping pattern on the second leg;

FIG. 3A is a top elevation close up view of a recess of the clip of FIG. 3;

FIG. 4 is another top elevation view of another clip, which is not an embodiment of the present invention, showing a first predetermined gripping pattern on the first leg and a second predetermined gripping pattern on the second leg with a rib therebetween;

FIG. 5 is another top elevation view of a clip, which does form an embodiment of the present invention;

FIG. 6 is still another top elevation view of another clip not forming part of the present invention;

FIG. 7 is yet another top elevation view of another clip, which does not form an embodiment of the present invention;

FIG. 7A shows a cross sectional view of the clip with a recess of the first leg aligned over a recess of the second leg;

FIG. 8 is a close up top view of a shaped recess of the clip;

FIG.8A is a cross sectional view of the shaped recess of Fig. 8 along line A-A.of Fig. 8;

FIG. 9 is a view of the compressed hemostatic clip occluding a vessel;

Fig. 10 is a view of a reverse diamond shape pattern of the recesses of the compressed hemostatic clip according to the present invention; and

Fig. 11 is a cross sectional view of the reverse diamond shape of Fig. 10.

### DETAILED DESCRIPTION OF THE EMBODIMENTS

As used in this specification and the appended claims, the singular forms "a," "an," and "the" include plural referents unless the context clearly dictates otherwise. It should be appreciated that these designation form no limitations on the present disclosure whatsoever.

The surgical hemostatic clip of the subject disclosure is applied to body tissue by an appropriate surgical clip applying apparatus. A surgical clip applying apparatus generally has structure to position the clip relative to the tissue to which it will be applied, and a mechanism to deform the clip, usually by compressing the clip at a preselected position thereof to clamp the tissue or vessel.

Some surgical instruments suitable for use in the application of the surgical hemostatic clips described herein are set forth in commonly assigned U.S. Pat. No. 4,509,518 to McGarry et al.; U.S. Pat. Nos. 5,084,057 and 5,100,420 to Green et al.; U.S. Patent No.: 5,269,792 to Kovac; U.S. Patent Application Serial No. 60/617,016; United States Patent Application Serial No.: 11/245,523 filed on October 7, 2005, United States Provisional Patent Application Serial No.: 60/617,104 filed on October 8, 2004 and United States Provisional Patent Application Serial No.: 60/617,016 filed on October 8, 2004.

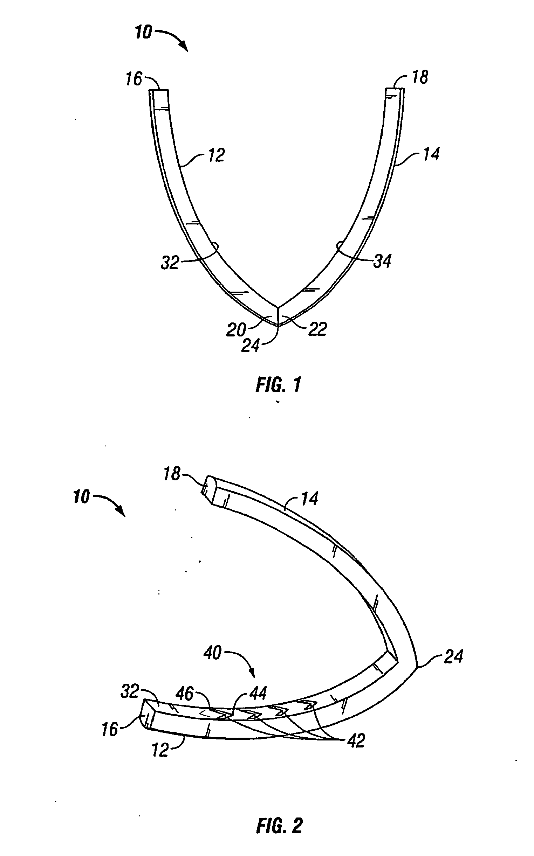

Referring now to FIG. 1, surgical hemostatic clip 10 includes a first leg 12 and a second leg 14. The first leg 12 has a distal end region 16. The second leg 14 has a distal end region 18. Each of the legs 12, 14 has respective proximal end regions 20 and 22 that connect the legs 12, 14 to one another. The proximal end regions 20 and 22 of each of the legs 12, 14 of hemostatic clip 10 are connected to one another forming an apex 24. The apex 24 of clip 10 has a generally V-shaped configuration as shown in Fig. 1. One skilled in the art should appreciate that the proximal end regions 20, and 22 may be connected differently to form a "U" shaped clip or another symmetrical or asymmetrical configuration. The clip 10 may have the form of the letter "U", V, or similar symmetric or asymmetric shapes resembling a broad "U" or V in cross profile or another shape such as one having an occluding portion and a compression portion. The occluding portion is one that can contact the tissue and/or vessel to occlude a vessel. The compression portion has a surface that can receive a compression force from an instrument in order to form the hemostatic clip 10 over the vessel to interrupt fluid flow through the vessel without damaging or harming the tissue or vessel. Various configurations for the compression portion and occluding portion are envisioned and the pattern of the hemostatic clip 10 may be used with various different clip geometries and the present disclosure is not limited to any specific clip geometry. Each of the legs 12 and 14 of hemostatic clip 10 define a respective tissue gripping surface on a lateral side of the hemostatic clip 10. It is envisioned that the desired tissue such as an artery, vessel or vein is clamped between the tissue gripping surfaces between the legs 12, 14 during application of the surgical hemostatic clip 10 for occlusion, or notably the obstruction or a closure of a passageway or vessel. Notably, leg 12 defines tissue gripping surface 32, while leg 14 defines tissue gripping surface 34.

Fig. 2 shows a perspective view of the clip 10 having a "V" shape; however it should be appreciated that instead of the "V" shape of Fig. 2, the clip 10 may have a "U" shape or another clip shape or desired geometry. Referring to Fig. 2, the tissue gripping surface 32 of the clip 10 has a first predetermined gripping pattern 40. As used herein, the term "gripping pattern" means any arrangement, structure or pattern that promotes exudation of tissue. The gripping pattern assists with the compressed clip 10 being retained on the vessel for occlusion purposes. As used herein, the term "tissue exudation" means the process upon which the tissue gradually moves, oozes or traverses into a recess, cavity, lateral surface, apex, distal end, chamfer, textured surface or structure of the clip to remain frictionally engaged thereon such that the clip remains on the desired tissue without damaging the vessel or tissue until physically removed or with degradable clips, until the clip disintegrates. One of ordinary skill in the art should appreciate that the clip 10 and gripping pattern 40 does not pierce tissue, and instead the clip 10 when deformed and in a compressed stated applies a compressive force to the outer surface of tissue in order to provide for occlusion of the tissue or vessel.

The predetermined gripping pattern 40 grasps and retains tissue for occlusion of a vessel in a manner that is particularly advantageous. The tissue is gripped and is retained on the clip 10 when the clip is compressed on tissue. The tissue will remain gripped on the clip 10 for a period of time, or until removed for occlusion during the entire surgical procedure.

In the example shown in Fig. 2, the clip 10 has the first predetermined gripping pattern 40 with a number of recesses 42. Each of the recesses 42 may have a shape or geometry that is particularly advantageous for gripping and retaining tissue. In this example, each recess 42 is generally triangular or "V" shaped and has an apex 44 and base portions 46. In another embodiment, the recesses 42 may be "U" or "C" shaped. In still another example, the recesses 42 may be orthogonal or have a slight curvature. The "V" shaped recesses 42 grip tissue when occluding and does not damage the occluded vessel, artery, vein or passageway. The V-shape of the recess 42 permits tissue exudation during the application of hemostatic clip 10 to tissue during compression of the clip 10. The exudation of tissue into recesses 42 inhibits movement of the hemostatic clip 10 relative to the applied vessel.

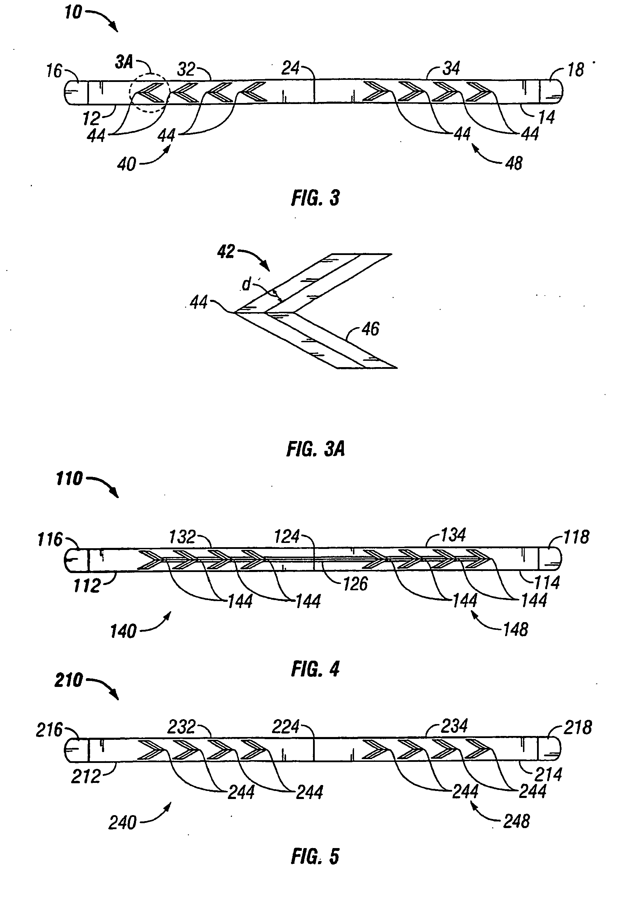

Referring now to Fig. 3, there is shown a top elevation view of the clip 10 showing the tissue gripping section 32 of the first leg 12 and the tissue gripping section 34 of the second leg 14. ln this example, the tissue gripping section 32 has first predetermined gripping pattern 40. The tissue gripping section 34 of the second leg 14 has a second predetermined gripping pattern 48. It should be appreciated that the first and second gripping patterns 40, 48 may be the same or may be different. Additionally, the tissue gripping sections 32, 34 each may have one or more different patterns thereon.

The first predetermined gripping pattern 40 has a number of "V" shaped recesses 42 in an optimal tissue gripping pattern. As shown, each of the "V" shaped recesses 42 has apex portion 44, the base portions 46, and a slight depth "d" as shown in an enlarged view of Fig. 3A. Referring again to Fig. 3, each apex portion 44 of the tissue gripping surface 32 points towards distal end 16 of the clip 10. Likewise, each apex portion 44 (of tissue gripping surface 34) points toward the distal end 18 of second leg 14. As shown each of the recesses 42 of the first predetermined gripping pattern 40 is in registration with each of the recesses 42 of the second predetermined gripping pattern 48 when the clip 10 has been deformed. Notably, the registration of the recesses 42 is advantageous as the orientation promote an overall or alignment between recesses 42 of the first leg 12 and the second leg 14 and is intended to further optimally inhibit movement of the hemostatic clip 10 relative to the vessel, artery or vein to which it is applied when the first leg 12 is compressed toward second leg 14 to deform clip 10.

As used herein, the term "in registration" or "registering" means that the recesses 42 of the tissue gripping surface 32 form a pattern have a leading edge, sidewall or substructure in the recess 42 that substantially aligns in location along the legs 12, 14 with another pattern of recesses 42 of the tissue gripping surface 34. The alignment promotes the tissue, vessel, or vein to contact the legs 12 and 14. The alignment allows the tissue to exude into the depth "d" of each of the recesses 42 and be frictionally engaged thereon for occlusion until removed. Thus, a superior benefit is achieved as the clip 10 provides that only a minimal (but optimal) amount of tissue gripping surfaces 32, 34 has a gripping feature thereon to promote exudation. This maximizes an amount of friction to retain the tissue on the clip 10. For the purposes of the instant clip 10, the term "in registration" or "in registering" does not necessarily mean that the recesses 42 of leg 12 and the recesses 42 of leg 14 are in an exact mirror image configuration. Instead, the recesses 42 overlie or overlap one another when leg 12 is compressed to leg 14. Here, the recess 42 of the first clip leg 12 overlie, or overlap with the recesses 42 of the second clip leg 14 when compressed while not strictly being in an exact mirror image configuration.

Referring now to Fig. 4, there is shown another top elevation view of another alternative clip 110 showing the tissue gripping section 132 of the first leg 112 and the tissue gripping section 134 of the second leg 114. The tissue gripping section 132 has the first predetermined gripping pattern 140 and the tissue gripping section 134 has the second predetermined gripping pattern 148. In this example, the first predetermined gripping pattern 140 has "V" shaped recesses 142. These recesses 142 include apex portions 144 which point toward the apex 124 of the clip 110. The second predetermined gripping pattern 142 has apex portions 144 which points towards distal end 118 of the second leg 114. Further, the clip 110 has a channel 126. The channel 126 extends opposite the apex 124 of the clip 110 or at a complementary location where the first leg 112 intersects the second leg 114. The channel 126 assists with the occlusion of the vessel by forcing the tissue between first leg 112 and second leg 114 to compress around and into the channel 126.

One known surgical clip is United States Patent No. 5,269,792 to Kovac, et al. (hereinafter "Kovac"). Kovac discloses at column 10, lines 35 though 37 that the surgical clip has a number of angled grooves extending from one edge of the clip to another edge of the clip. Kovac discloses that the angled grooves improve the grip of the clip on the tissue. The present clip 110 of Fig. 4 is advantageous over Kovac as the recesses 142 of the first clip leg 112 cooperate with the recesses 142 of the second clip leg 114 when the clip 110 is compressed to form a two dimensional polygonal pattern.

Referring now to Fig. 5, there is shown another top elevation view of clip 210 according to the present invention showing the tissue gripping section 232 of the first leg 212 and the tissue gripping section 234 of the second leg 214. In this embodiment, the tissue gripping section 232 has a first predetermined gripping pattern 240 and the tissue gripping section 234 has a second predetermined gripping pattern 248 with the first predetermined gripping pattern 240 having the apex portion 244. Each apex portion 244 of each recess 242 points towards the apex 224 of clip 210. Likewise, the second predetermined gripping pattern 248 has each apex portion 244 of each recess 242 pointing toward the distal end 218 of the second leg 214. Clip 210 is similar to clip 110 of Fig. 4, but is formed without any channel 126 as shown in Fig. 5. As can be understood, the clip 210 will be positioned over the desired vessel, vein or artery. The clip 210 will then be compressed to occlude the desired vessel, vein or artery using the relevant surgical instrument such as a clip applier.

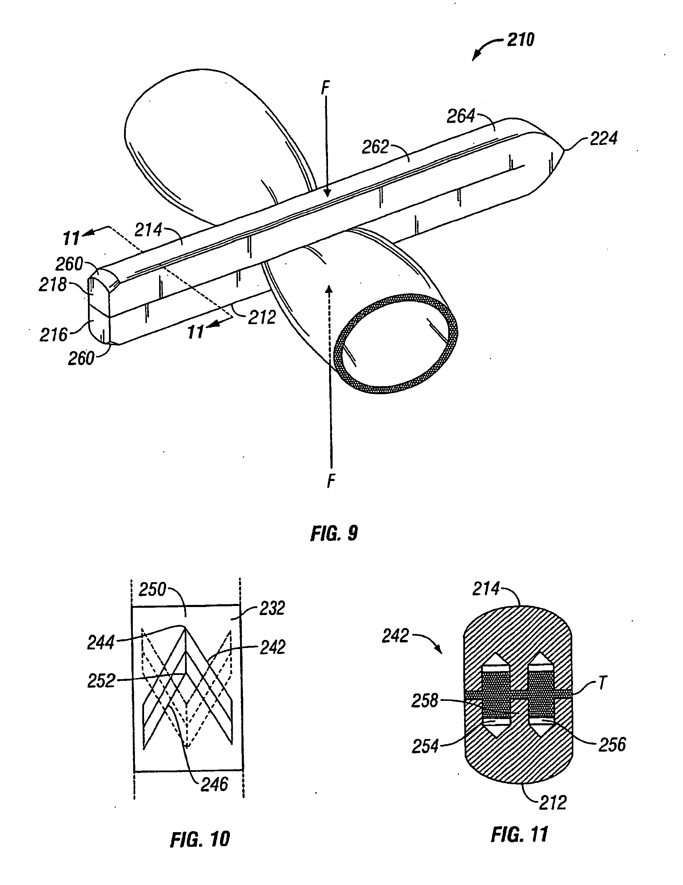

The recesses 242, on the first clip leg 212 are disposed over and in registration with the recesses 242 of the second clip leg 214. When compressed, the recesses 242 of the first clip leg 212 and the second clip leg 214 together form a two dimensional polygonal or diamond shaped pattern with the tissue being disposed therebetween as illustrated in Fig. 10. The tissue exudes into the recesses 242 of each clip leg 212, 214, and is firmly retained therein. As stated, in this manner, an apex portion 244 of the one recess 242 on the first clip leg 212 is disposed one hundred and eighty degrees from a corresponding apex portion 244 of the second clip leg 214 when clip 210 is compressed.

Referring now to Fig. 10, there is shown a close up view of the clip 210 having the first leg 212 compressed toward the second leg 214. As can be understood from the figure, the leg 212 is compressed toward the leg 214 so the clip 210 can be applied to the desired tissue. Here, the recess 242 of the first clip leg 212 is advantageously disposed over the recesses 242 of the second clip leg 214 when clip 210 is compressed. Recess 242 of leg 214 is shown in phantom broken lines for illustration purposes only. Here, in Fig. 10, the recesses 242 of the first clip leg 212 are in registration with the recesses 242 of the second clip leg 214 when clip 210 is compressed as shown in phantom broken lines.

Together, the recesses 242 of the first clip leg 12 which are disposed over the recesses 242 of the second clip leg 214 forms a two dimensional diamond shaped pattern with the tissue being disposed therebetween as shown in Fig. 10 with the recesses 242 if second leg 214 shown in phantom lines. In this manner, an apex portion 244 of the one recess 242 on the first clip leg 212 is disposed one hundred and eighty degrees from apex portion 244 of the recess 242 of the second clip leg 214 when the clip 210 of Fig. 5 is compressed.

Referring to Fig. 11, there is shown a cross sectional view of the compressed clip 10 of Figs. 5 and 10 with the first leg 212 compressed over the second leg 214. The compressed clip 210 has the diamond shaped pattern (with the recess 242 of the first clip leg 212 disposed over the recesses 242 of the second clip leg 214). As can be seen from the cross sectional view, the tissue as illustrated by reference letter "T" traverses into the recess 42 of the first clip leg 12 and into the recess 242 of the second clip leg 214 with the apex portion 244 (of the one recess 242 on the first clip leg 212) disposed one hundred and eighty degrees from another apex portion 244 of the recess 242 of the second clip leg 214. The compressed clip 210 has a two dimensional diamond shaped pattern when the recesses 242 to overlap to promote tissue exudation. The compressed clip 210 having a two dimensional diamond shaped pattern when the recesses overlap firmly holds the clip 10 on the tissue to block the flow of fluid therethrough.

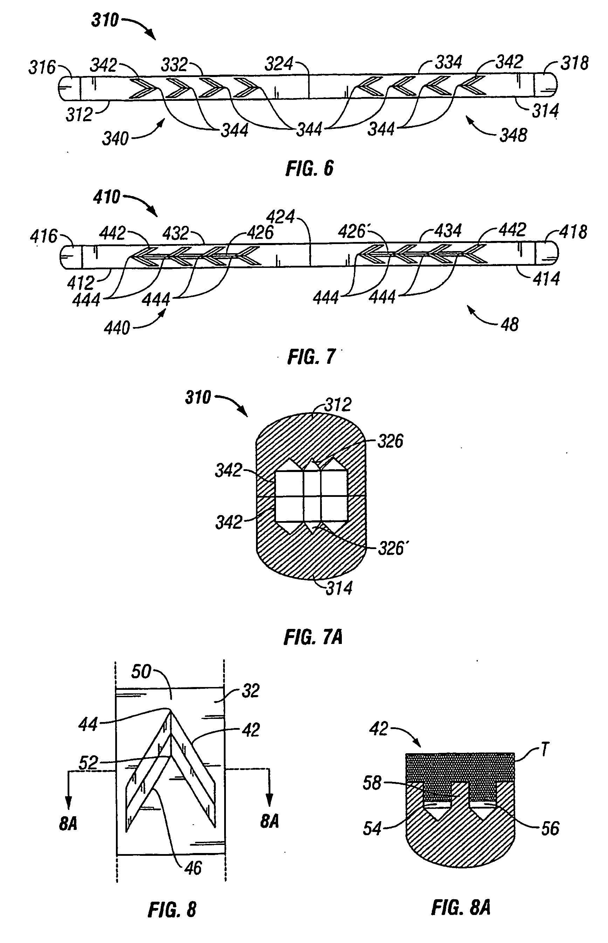

Referring now to Fig. 6, there is shown another top elevation view of another non-claimed example of the clip 310 showing the tissue gripping section 332 of the first leg 312 and the tissue gripping section 334 of the second leg 314. In this example, the first predetermined gripping pattern 340 has the apex portion 344 of each of the recesses 342 pointing toward the apex 324 of the clip 310. Likewise, the second predetermined gripping pattern 348 has the apex portion 344 of each of the recesses 342 also pointing toward the apex 324 of the clip 310.

Referring now to Fig. 7, there is shown another top elevation view of the clip 410 of Fig. 6 showing the tissue gripping section 432 of the first leg 412 and the tissue gripping section 434 of the second leg 414. Here, the first predetermined gripping pattern 440 is reversed with the apex portion 444 of each of the recesses 442 pointing toward distal end 416 of the first leg 412. Also, the second predetermined gripping pattern 442 is made with each apex portion 444 pointing to the apex 424 of the clip 410. The clip 410 in this example further has the channel 426 that is a segmented channel pattern. The channel 426 on the first leg 412 does not connect with the channel 426' on the second leg 414. In contrast, the channel 426 connects the apex portion 444 of each recess 442 with one another then terminates. Further, the channel 426' on the second leg 414 connects each apex portion 444 of each recess 442 with one another. This permits tissue exudation on the recesses 442 and channel 426 of the first clip leg 412 and tissue exudation in the recesses 442 and channel 426' of the second clip leg 414. Referring now to a cross sectional view of the clip of Fig. 6 showing the first leg 312 compressed to the second leg 314 (Fig. 7A), one can see that an interior space of recess 342 of the first leg 312 substantially align with another recess 342 of the second leg 314. In this manner, the tissue can enter each recess 342 for maximum tissue exudation and to promote occlusion of the desired vessel. In the example shown in Fig. 7, the channel 426 of the first leg 412 also substantially aligns with the channel 426' on the second leg 414 for promotion of tissue exudation and to assist retaining the clip on the desired tissue.

Fig. 8 shows a close up top view of one recess 42 of the clip 10 of Figs. 1 through 3. As can be understood, the recess 42 is separated from another adjacent recess by a distance. The leg 12 has an outer surface 50 that is generally flat. Each of the recesses 42 has a "V" shape. Each recess 42 is made with an apex portion 44. Closely adjacent to the apex portion 44 of the recess 42 is a leading edge 52. The leading edge 52 is pointed to a tip and in this example assists with gripping the exuded tissue that enters the recess 42 when the clip 10 is compressed. This recess 42 is disposed on the tissue gripping surface 32 of the first leg 12; however the recess 42 may be disposed in other locations. In this example, the leading edge 52 is pointed in a direction parallel to a lateral edge of the clip 10, however, the leading edge 52 may be formed so as to point in other directions angled from the lateral edge of the clip 10 or even be formed perpendicular to the lateral edge of the clip 10 or even at a number of intermediate angles therebetween depending on the recesses 42 of the other leg. Various configurations are possible and one skilled in the art should appreciate that the clip 10 may be formed with the leading edge 52 pointing in a number of different locations so long as the recesses 42 of one leg 12 are in registration with recesses 42 of another leg 14 to form the two dimensional polygonal or diamond shaped pattern as shown in Fig. 10. Various configurations are possible.

Referring to a cross sectional view of one recess 42 as shown in Fig. 8A along line A-A of Fig. 8, the recess 42 has a first portion 58 and a first and second sub recess 54, 56. The first portion 58 splits the first and second sub recesses 54, 56. When the clip 10 is compressed, the relevant tissue section shown as reference letter "t" will ooze, exude or otherwise slightly traverse into the first and second sub recesses 54, 56 of the recess 42 and be retained therein by the leading edge 52 and by a frictional engagement of the lateral side walls of the recess 42. The lateral side walls of the recess 42 may further optionally overhang over the first and second sub recesses 54, 56 to assist with tissue exudation. Likewise, the first portion 58 of the leg 12 may further have a surface treatment to further assist with clip retention.

With reference to FIG. 1, the surgical hemostatic clip 10 of the subject disclosure may be of any dimension suitable for application to vessels and body tissue. In one embodiment, the length of the clip 10 is about 7.95 millimeters and the width of the clip 10 from an outer surface of the first leg 12 to an outer surface of the second leg 14 is about 4.57 millimeters, and the surgical clip 10 is intended to be used with a five millimeter trocar. One of ordinary skill in the art will recognize that other dimensions can also be used, and the dimensions of the clip 10 may be modified to various other dimensions to fit various clip appliers, trocars, tissue, vessels, arteries or other surgical procedures.

The structures described herein may be formed in clip 10 by molding or by applying an appropriate stamping force to the faces of legs 12 and 14. Alternatively, structures may be formed by machining clip 10 or by other known metal or polymer processing techniques. For example, clip 10 may be molded with the structures formed therein. The surgical hemostatic clip 10 of the present disclosure may be fabricated from any biocompatible material including stainless steel, titanium, and tantalum, as well as plastic materials including biocompatible polymers, or a combination of materials thereof.

Referring now to Fig. 9, there is shown the clip 10 of the example of Figs. 1 through 3 in a compressed state and over a vessel such as an artery or vein for occlusion. One skilled in the art should appreciate that no fluid traverses through the vessel when the clip 10 is compressed over the vessel. Notably, the first and the second predetermined gripping patterns 40, 48 grip and retain an amount of tissue therein and retain the clip 10 on the vessel for the entire surgical procedure or as desired until physically removed.

Fig. 9 shows a view of the first distal end 16 and the second distal end 18 of the legs 12, 14. Each of the ends has a chamfer 60. The chamfer 60 is on an outer surface of distal ends 16, 18 of the clip 10. The clip 10 further may have a textured feature 62. The textured feature 62 is on an outer surface 64 of the clip 10. Textured feature 62 assists with retaining the clip 10 in the clip applier 10. Textured feature 62 may be a surface roughness, a number of striations, a number of protuberances, a rough or grainy surface quality or any other surface quality that increases a coefficient of friction to the outer surface 62, and assists with manipulating the clip 10, and compressing the clip 10 to apply the clip 10 to body tissue.

Referring to Fig. 9, the method of making the clip 10 provides that the clip 10 has an improved surface treatment. The method 70 has the following steps. First, the clip 10 is stamped, forged or molded and formed into a generally desired shape as disclosed in Figs 1 through 11. Various shapes are possible and within the scope of the present disclosure. Accordingly, at step 72, the clip 10 is stamped, forged, molded, or machined into the desired "V" or "U" shape. The clip 10 is a metallic material.

In one embodiment, the clip 10 is an ASTM F67 grade 1 Titanium. However, the clip 10 may be other materials or in another embodiment may be an alloy, steel, metal, another grade of titanium, or another similar biocompatible or suitable implantable material. Thereafter, the method has the step of heating the clip 10. The clip 10 is, in one embodiment, heated in a vacuum. The method also has the step of slow cooling the clip 10 to strengthen and harden the clip 10 to change a material characteristic of the clip 10. The cooling of the clip 10 is performed in order to stress relieve and change the clip hardness and Rockwell strength of the clip 10. Various cooling parameters in order to modulate the Rockwell strength of the clip 10 are possible and within the scope of the present disclosure.

At step 74, the clip 10 is heated to a desired temperature. The desired temperature is in one example 1,275 degrees Fahrenheit (690.6°C) for a predetermined time period. In one embodiment, the time period may be one hour, however other suitable time periods are possible. Alternatively, the desired temperature is in another embodiment 1,250 degrees Fahrenheit (677°C). However, this desired temperature is non-limiting and any acceptable temperature may used in order to heat the clip to a temperature where annealing is possible depending on the material of the clip 10. For the purposes of the temperature range given above the clip 10 is made from titanium. The clip 10 is heated in a suitable furnace, oven or other suitable device or heating apparatus. Thereafter, the method 70 proceeds to step 76. The heated clip is then soaked for a period of time. The clip 10 is soaked in an inert gaseous substance. In one example, the clip 10 is soaked in an inert Argon gas for a predetermined amount of time or in another similar acceptable soaking substance. Thereafter, the method 70 proceeds to step 78. At step 78, the heated clip 10 contacts an argon gas. The clip 10 is first placed and heated in a suitable compartment. After the clip is heated, the clip is then soaked with the Argon gas. The argon gas controls and actively modulates or restructures the crystal size and alignment of the titanium of the clip in proportion to the argon gas as the titanium cools in order to control the microstructure of the clip 10. The heated clip 10 is cooled at a uniform rate. In one embodiment, the clip 10 may be furnace or air cooled.

Thereafter, the method 70 proceeds to step 80. At step 80, the clip is removed from the compartment and tested for hydrogen content. The analysis is nondestructive. The analysis measures the sample and the results are independent of the specific chemical form of the hydrogen or related hydrogen compounds present. At step 80, the clip 10 is tested using a cold neutron prompt gamma ray activation analysis apparatus (CNPGAA) or similar machine. Alternatively, the clip 10 may be tested using an X-ray diffraction device. Thereafter, the method further has the steps of machining a first plurality of recesses 42 with each having a first apex portion in a first leg 12 of the clip 10, and machining a second plurality of recesses 42 having a second apex portion in a second leg 14 of the clip. When the clip is compressed the second plurality of recesses are in registration with the first plurality of recesses. The first apex portion is disposed one hundred and eighty degrees from the second apex portion to form when compressed a two dimensional diamond shaped pattern as shown in Fig. 10. Thereafter, the method also has a tumbling process. The tumbling process involves placing the clips in another sealed tumbling compartment, and moving the compartment to impart kinetic energy to the clips for a predetermined period of time. The tumbling compartment may have another rigid resilient substance therein. The clips contact an inner surface of the tumbling compartment or the substance. The repeated contact gives the clip one or more holding surfaces to promote retention on tissue. Further, the repeated contact gives an exterior of the clips 10 a predetermined textured finish conducive for surgery.

## Claims

1. A surgical clip (10) for a clip applier, the surgical clip comprising:
a first leg (12) connected to a second leg (14) at a first location (24);
wherein the first leg is separated from the second leg by a predetermined distance at a second location spaced from the first location, the first leg forming a first distal end (16) and the second leg forming a second distal end (18), the first leg having a first tissue gripping surface and the second leg having a second tissue gripping surface;
wherein the first tissue gripping surface (32) has a first recess (42) on the first leg, and the second tissue gripping surface (34) has a second recess (42) on the second leg;
wherein the first recess consists of an apex portion (44) and a first and a second distal leg portion (46) extending from the apex portion, wherein the apex portion and each leg portion of the first recess is located along the first tissue gripping surface;
wherein the second recess consists of an apex portion (44) and a first and a second distal leg portion (46) extending from the apex portion, wherein the apex portion and each leg portion of the second recess is located along the second tissue gripping surface; and **characterised by**:
when the clip is compressed the first clip leg is compressed to the second clip leg, the apex portion of the first clip leg and the apex portion of the second leg point toward opposite directions and the first recess is compressed towards the second recess such that the leg portions of the first recess overlap with the leg portions of the second recess to form a two-dimensional polygonal pattern.

2. The surgical clip of claim 1, wherein the first leg has a plurality of the first recesses (42) and the second leg has a plurality of the second recesses (42), each of the first recesses registering with another of the second recesses when the first clip leg is compressed toward the second clip leg to form a two dimensional polygonal pattern along the first and second tissue gripping surfaces,
wherein the first recesses each consist of an apex portion (44) and a first and a second distal leg portion (46) extending from the apex portion, wherein the apex portion and each leg portion of each of the first recesses is located along the first tissue gripping surface,
wherein the second recesses each consist of an apex portion (44) and a first and a second distal leg portion (46) extending from the apex portion, wherein the apex portion and each leg portion of each of the second recesses is located along the second tissue gripping surface,
wherein when the first clip leg is compressed to the second clip leg, the apex portions of the first clip leg and the apex portions of the second leg point toward opposite directions such that the leg portions of the first recesses overlap with the leg portions of corresponding second recesses.

3. The surgical clip of any one of the preceding claims, further including a grip feature (62) on an outer clip leg surface (64).

4. The surgical clip of any one of the preceding claims, wherein the surgical clip is a hemostatic clip and is made from a material selected from stainless steel, a polymer, titanium, a biocompatible material, and any combinations thereof.

5. The surgical clip of any one of the preceding claims, wherein the first recess and the second recess are contained laterally within the respective first and second tissue gripping surfaces.

## Patentansprüche

1. Chirurgische Klammer (10) für einen Klammerapplikator, wobei die chirurgische Klammer aufweist:
ein erstes Bein (12), das mit einem zweiten Bein (14) an einer ersten Stelle (24) verbunden ist;
wobei das erste Bein von dem zweiten Bein durch einen festgelegten Abstand an einer von der ersten Stelle beabstandeten zweiten Stelle getrennt ist, das erste Bein ein erstes distales Ende (16) ausbildet und das zweite Bein ein zweites distales Ende (18) ausbildet, das erste Bein eine erste Gewebegreiffläche aufweist und das zweite Bein eine zweite Gewebegreiffläche aufweist;
wobei die erste Gewebegreiffläche (32) eine erste Aussparung (42) an dem ersten Bein aufweist und die zweite Gewebegreiffläche (34) eine zweite Aussparung (42) an dem zweiten Bein aufweist;
wobei die erste Aussparung aus einem Scheitelabschnitt (44) und einem ersten und einem zweiten distalen Beinabschnitt (46) besteht, die sich von dem Scheitelabschnitt erstrecken, wobei der Scheitelabschnitt und jeder Beinabschnitt der ersten Aussparung entlang der ersten Gewebegreiffläche angeordnet sind;
wobei die zweite Aussparung aus einem Scheitelabschnitt (44) und einem ersten und einem zweiten distalen Beinabschnitt (46) besteht, die sich von dem Scheitelabschnitt erstrecken, wobei der Scheitelabschnitt und jeder Beinabschnitt der zweiten Aussparung entlang der zweiten Gewebegreiffläche angeordnet sind; und **dadurch gekennzeichnet, dass**:
wenn die Klammer gedrückt wird, das erste Klammerbein zu dem zweiten Klammerbein gedrückt wird, der Scheitelabschnitt des ersten Klammerbeins und der Scheitelabschnitt des zweiten Klammerbeins in entgegengesetzte Richtungen zeigen und die erste Aussparung so in Richtung der zweiten Aussparung gedrückt wird, dass die Beinabschnitte der ersten Aussparung sich mit den Beinabschnitten der zweiten Aussparung überlappen, um ein zweidimensionales Polygonmuster auszubilden.

2. Chirurgische Klammer nach Anspruch 1, bei der das erste Bein eine Vielzahl der ersten Aussparungen (42) aufweist und das zweite Bein eine Vielzahl der zweiten Aussparungen (42) aufweist, wobei jede der ersten Aussparungen eine andere der zweiten Aussparungen erfasst, wenn das erste Klammerbein in Richtung des zweiten Klammerbeins gedrückt wird, um ein zweidimensionales Polygonmuster entlang der ersten und zweiten Gewebegreifflächen auszubilden,
wobei die ersten Aussparungen jeweils aus einem Scheitelabschnitt (44) und einem ersten und einem zweiten distalen Beinabschnitt (46) bestehen, die sich von dem Scheitelabschnitt erstrecken, wobei der Scheitelabschnitt und jeder Beinabschnitt einer jeden der ersten Aussparungen entlang der ersten Gewebegreiffläche angeordnet sind,
wobei die zweiten Aussparungen jeweils aus einem Scheitelabschnitt (44) und einem ersten und einem zweiten distalen Beinabschnitt (46) bestehen, die sich von dem Scheitelabschnitt erstrecken, wobei der Scheitelabschnitt und jeder Beinabschnitt einer jeden der zweiten Aussparungen entlang der zweiten Gewebegreiffläche angeordnet sind,
wobei wenn das erste Klammerbein zu dem zweiten Klammerbein gedrückt wird, die Scheitelabschnitte des ersten Klammerbeins und die Scheitelabschnitte des zweiten Klammerbeins in entgegengesetzte Richtungen zeigen, sodass sich die Beinabschnitte der ersten Aussparungen mit den Beinabschnitten der jeweiligen zweiten Aussparungen überlappen.

3. Chirurgische Klammer nach einem der vorstehenden Ansprüche, des Weiteren mit einem Griffmerkmal (62) auf einer äußeren Klammerbeinfläche (64).

4. Chirurgische Klammer nach einem der vorstehenden Ansprüche, wobei die chirurgische Klammer eine hämostatische Klammer ist und aus einem Material, ausgewählt aus rostfreiem Stahl, einem Polymer, Titan, einem biokompatiblen Material und beliebigen Kombinationen davon, hergestellt ist.

5. Chirurgische Klammer nach einem der vorstehenden Ansprüche, bei der die erste Aussparung und die zweite Aussparung lateral in der jeweiligen ersten und zweiten Gewebegreiffläche enthalten sind.

## Revendications

1. Pince chirurgicale (10) pour un applicateur de pince, la pince chirurgicale comprenant:
une première patte (12) raccordée à une seconde patte (14) à un premier emplacement (24);
dans laquelle la première patte est séparée de la seconde patte par une distance prédéterminée à un second emplacement espacé du premier emplacement, la première patte formant une première extrémité distale (16) et la seconde patte formant une seconde extrémité distale (18), la première patte ayant une première surface de préhension de tissu et la seconde patte ayant une seconde surface de préhension de tissu;
dans laquelle la première surface de préhension de tissu (32) a un premier évidement (42) sur la première patte, et la seconde surface de préhension de tissu (34) a un second évidement (42) sur la seconde patte;
dans laquelle le premier évidement se compose d'une partie de sommet (44) et une première et une seconde partie de patte distale (46) s'étendant à partir de la partie de sommet, dans laquelle la partie de sommet et chaque partie de patte du premier évidement sont positionnées le long de la première surface de préhension de tissu;
dans laquelle le second évidement se compose d'une partie de sommet (44) et d'une première et d'une seconde partie de patte distale (46) s'étendant à partir de la partie de sommet, dans laquelle la partie de sommet et chaque partie de patte du second évidement sont positionnées le long de la seconde surface de préhension de tissu; et **caractérisée par**:
lorsque la pince est comprimée, la première patte de pince est comprimée sur la seconde patte de pince, la partie de sommet de la première patte de pince et la partie de sommet de la seconde patte de pince pointent vers des directions opposées et le premier évidement est comprimé vers le second évidement de sorte que les parties de patte du premier évidement chevauchent sur les parties de patte du second évidement, afin de former un modèle polygonal bidimensionnel.

2. Pince chirurgicale selon la revendication 1, dans laquelle la première patte a une pluralité de premiers évidements (42) et la seconde patte a une pluralité de seconds évidements (42), chacun des premiers évidements s'alignant avec un autre des seconds évidements lorsque la première patte de pince est comprimée vers la seconde patte de pince afin de former un modèle polygonal bidimensionnel le long des première et seconde surfaces de préhension de tissu,
dans laquelle les premiers évidements se composent chacun d'une partie de sommet (44) et d'une première et d'une seconde partie de patte distale (46) s'étendant à partir de la partie de sommet, dans laquelle la partie de sommet et chaque partie de patte de chacun des premiers évidements est positionnée le long de la première surface de préhension de tissu,
dans laquelle les seconds évidements se composent chacun d'une partie de sommet (44) et d'une première et d'une seconde partie de patte distale (46) s'étendant à partir de la partie de sommet, dans laquelle la partie de sommet et chaque partie de patte de chacun des seconds évidements est positionnée le long de la seconde surface de préhension de tissu,
dans laquelle, lorsque la première patte de pince est comprimée sur la seconde patte de pince, les parties de sommet de la première patte de pince et les parties de sommet de la seconde patte pointent vers des directions opposées de sorte que les parties de patte des premiers évidements chevauchent sur les parties de patte des seconds évidements correspondants.

3. Pince chirurgicale selon l'une quelconque des revendications précédentes, comprenant en outre une caractéristique de préhension (62) sur une surface de patte de pince externe (64).

4. Pince chirurgicale selon l'une quelconque des revendications précédentes, dans laquelle la pince chirurgicale est une pince hémostatique et est réalisée à partir d'un matériau choisi parmi l'acier inoxydable, un polymère, du titane, un matériau biocompatible et l'une quelconque de leurs combinaisons.

5. Pince chirurgicale selon l'une quelconque des revendications précédentes, dans laquelle le premier évidement et le second évidement sont contenus latéralement à l'intérieur des première et seconde surfaces de préhension de tissu respectives.
